# EUROPEAN PATENT APPLICATION

(11) **EP 4 116 296 A1**
(43) Date of publication of application: **11.01.2023**
(21) Application number: 21765519.0
(22) Date of filing: 02.03.2021
(51) Int. Cl.: C07D 277/50, A61K 31/426, A61P 31/12

(54) **ANTI-RNA VIRUS DRUG AND APPLICATION THEREOF**

(30) Priority: 02.03.2020 CN 202010136724
(71) Applicant: East China University of Science and Technology, Shanghai 200237 (CN); Wuhan University, Hubei 430072 (CN)
(72) Inventor: LI, Honglin, Shanghai 200237 (CN); XU, Ke, Wuhan, Hubei 430072 (CN); LI, Shiliang, Shanghai 200237 (CN); ZHU, Lili, Shanghai 200237 (CN); ZHAO, Zhenjiang, Shanghai 200237 (CN)
(74) Representative: reuteler & cie SA
(86) International application number: PCT/CN2021/078743
(87) International publication number: WO 2021/175224

(57) **Abstract**

An application of a novel thiazole derivative in the treatment of an RNA virus infection and the preparation of a drug for treating an RNA virus infection. Specifically, the present invention relates to a use of a compound represented by the following formula I and a pharmaceutical composition containing a compound represented by the following formula I in treating an RNA virus infection and preparing a drug for treating a coronavirus infection.

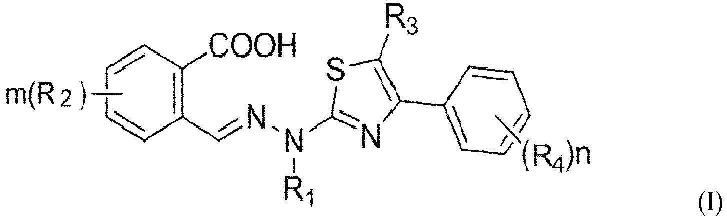

## Description

### Technical field

The invention relates to the field of medicinal chemistry; and in particular, the invention relates to the use of novel thiazole derivatives in the preparation of a medicament for treating RNA virus infection and in the treatment of virus infection.

### Background

A virus uses a host cell to complete its own reproduction, and infects the host cell through five processes, adsorption, invasion, replication, maturation and release, which seriously affects human health.

Coronaviruses are a class of enveloped single-stranded positive-stranded RNA viruses. In 2012, coronaviruses are classified by the International Committee on Taxonomy of Virology into four categories: α, β, γ and δ. A total of 7 coronaviruses have been found that can infect humans, namely human coronavirus 229E (HCoV-229E), human coronavirus NL63 (HCoV-NL63), human coronavirus OC43 (HCoV-OC43), Hong Kong type I human coronavirus (HCoV-HKU1), severe acute respiratory syndrome coronavirus (SARS-CoV), Middle East respiratory syndrome coronavirus (MERSCoV), and the new coronavirus (2019-nCoV) occurring in Wuhan. Among them, SARS-CoV, MERS-CoV and 2019-nCoV are the currently discovered highly pathogenic coronaviruses for human, which have brought serious harm to human health.

At present, the prevention and treatment of viral diseases mainly rely on vaccines and drugs. The vaccine itself has certain limitations: the immunization rate is low, so that it is difficult to generate effective herd immunity; it is difficult to generate effective immunity for high-risk groups, such as the elderly and immunocompromised people; it is necessary to continuously develop new vaccines when the virus continues to mutate due to the lack of post-translational correction mechanism of viral RNA polymerase, and it is also difficult to produce a sufficient number of new vaccines in a short period of time in the early stage of rapid epidemics.

Antiviral drug research remains a hot topic in the field of antiviral research. Commonly used antiviral drugs mainly include chemical drugs and herbal medicines. Different antiviral drugs have different mechanisms of action. In the early stage of virus infection, antiviral drugs fight against the virus by inhibiting the adsorption and invasion of the virus, and blocking the binding of the virus envalop to the receptors on the body's cell surface. When the virus enters the cell, antiviral drugs can achieve antiviral effects by inhibiting the replication, transcription and protein synthesis of viral nucleic acid. After the assembly of virus particle is complete, another mode of action of antiviral drugs is to inhibit virus release. In addition, under the condition that a body's resistance is reduced and the virus easily invades the host, and antiviral drugs can also fight against the virus by enhancing the body's resistance.

Antiviral drugs include two ion channel inhibitors: amantadine, two neuraminidase inhibitors, oseltamivir and inhaled zanamivir. However, most influenza viruses are resistant to both amantadine and oseltamivir. For example, the seasonal H1N1 virus in the United States in 2009 developed resistance to oseltamivir, while almost all H3N2 viruses are resistant to amantadine.

There is also no specific drug for the outbreak of 2019-nCoV. Currently, several broad-spectrum antiviral drugs are recommended, including lopinavir/ritonavir and some traditional medicine preparations. In the face of the current emergency situation, the research and development of new antiviral drugs is necessary.

### Summary of the invention

The purpose of the present invention is to provide a novel, broad-spectrum compound with anti-coronavirus infection activity, so as to be used as a therapeutic drug for coronavirus infection.

In the first aspect, the present invention provides the use of a compound of formula I, or a pharmaceutically acceptable salt thereof, in the preparation of a drug against RNA virus infection: 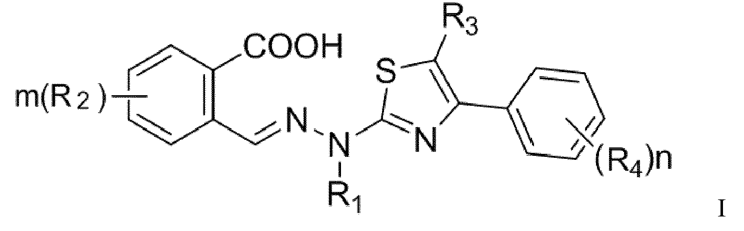 wherein,
R₁ is selected from: H, a substituted or unsubstituted C1-C6 alkyl, C3-C6 cycloalkyl;
R₂ is independently selected from: H, a halogen, substituted or unsubstituted C1-C6 alkyl, substituted or unsubstituted C1-C6 alkoxy, CN, NO₂, hydroxyl, NR^{a}R^{b};
R^{a} and R^{b} can be independently selected from H or a C1-C6 alkyl;
R₃ is selected from: H, a substituted or unsubstituted C1-C6 alkyl;
R₄ is independently selected from: H, a halogen;
m is an integer from 0 to 4;
n is an integer from 0 to 5.

In a specific embodiment, the RNA viruses include, but not limited to, coronaviruses, such as severe acute respiratory syndrome coronavirus (SARS-CoV), Middle East Respiratory Syndrome Coronavirus (MERSCoV), and 2019 novel coronavirus (2019-nCoV) ); Ebola virus, bunya virus, avian influenza H9N2, H1N1, H7N9, arena virus, rabies virus, hepatitis C HCV, hepatitis B HBV, human immunodeficiency virus HIV-1, herpes simplex virus HSV-1, HSV-2, etc.

In a preferred embodiment, the RNA viruses include, but not limited to: severe acute respiratory syndrome coronavirus (SARS-CoV), Middle East Respiratory Syndrome Coronavirus (MERS-CoV) and 2019 novel Coronavirus (2019-nCoV), Ebola virus, fever with thrombocytopenia syndrome virus (sftsv), avian influenza virus H9N2, H1N1.

In a specific embodiment, the compound is shown in formula II: 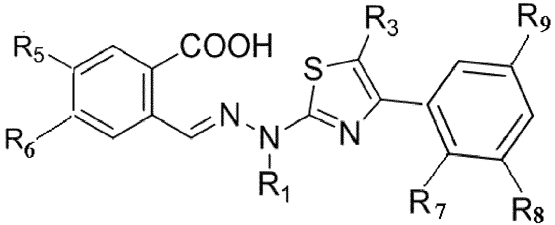 wherein,
R₅ and R₆ are independently selected from: H, a halogen, substituted or unsubstituted C1-C6 alkyl, substituted or unsubstituted C1-C6 alkoxy, hydroxyl, NH₂;
R₇, R₈ and R₉ are independently selected from: H, a halogen;
R₁ and R₃ are described as above.

In a specific embodiment, in the formula
R₁ is selected from: H, a C1-C6 alkyl;
R₃ is selected from: H, a substituted or unsubstituted C1-C6 alkyl;
R₅ and R₆ are independently selected from: H, a halogen, unsubstituted or halogen-substituted C1-C3 alkyl;
R₇ and R₈ are independently selected from: H, a halogen;
R₉ is H.

In a preferred embodiment, in the formula, R₁ is selected from H or methyl.

In a specific embodiment, the compound shown in formula I or a pharmaceutically acceptable salt thereof is a compound selected from the following group or a pharmaceutically acceptable salt thereof:

| No. | Structure of compound | No. | Structure of compound |
|---|---|---|---|
| 1 | 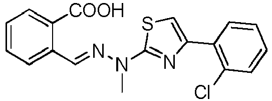 | 2 | 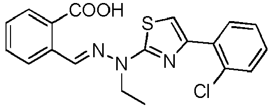 |
| 3 | 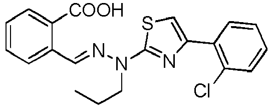 | 4 | 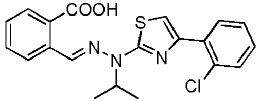 |
| 5 | 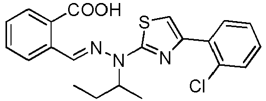 | 6 | 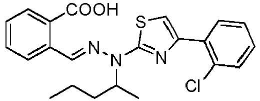 |
| 7 | 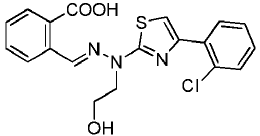 | 8 | 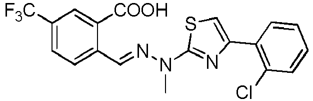 |
| 8-1 | 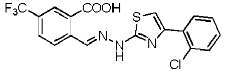 | 9 | 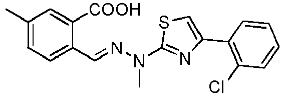 |
| 9-1 | 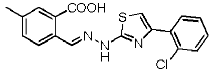 | 10 | 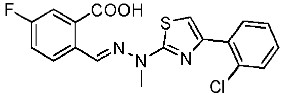 |
| 10-1 | 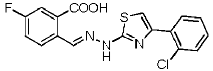 | 11 | 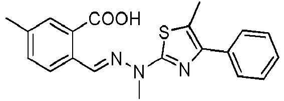 |
| 12 | 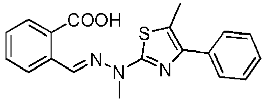 | 12-1 | 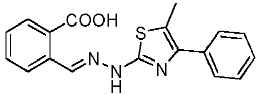 |
| 13 | 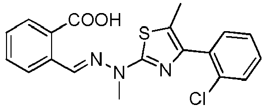 | 14 | 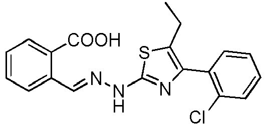 |
| 15 | 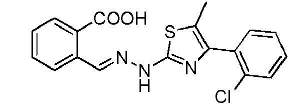 | 16 | 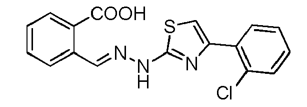 |
| 17 | 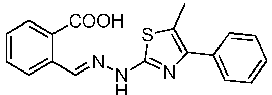 | 18 | 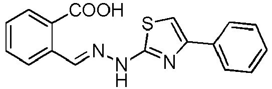 |
| 19 | 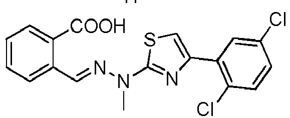 | 20 | 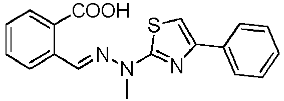 |
| 21 | 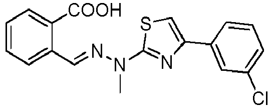 | 22 | 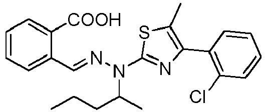 |
| 23 | 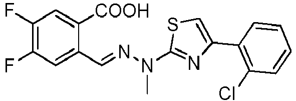 | 23-1 | 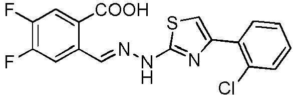 |
| 24 | 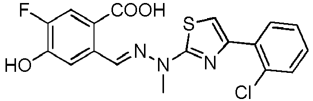 | 25 | 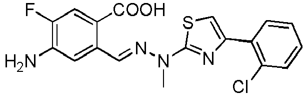 |
| 52 | 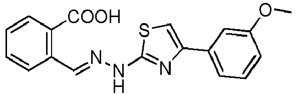 | 53 | 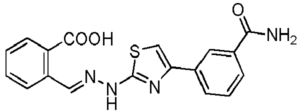 |
| 54 | 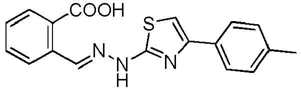 | 55 | 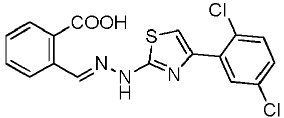 |
| 56 | 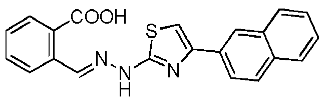 | 57 | 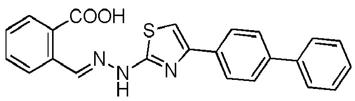 |
| 58 | 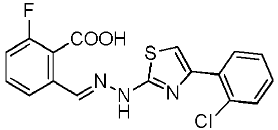 | 59 | 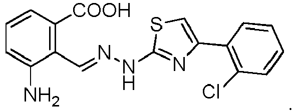 |

In a specific embodiment, the compound is a compound selected from the following group:

| No. | Structure of compound | No. | Structure of compound |
|---|---|---|---|
| 1 | 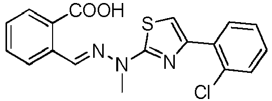 | 2 | 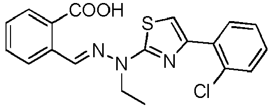 |
| 8 | 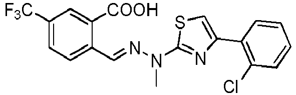 | 8-1 | 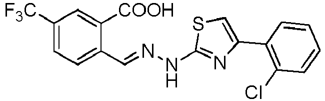 |
| 9 | 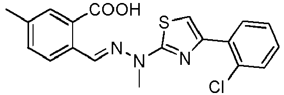 | 9-1 | 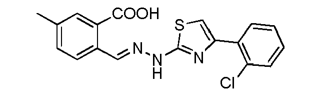 |
| 10 | 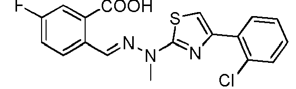 | 10-1 | 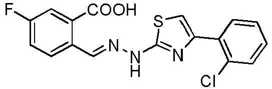 |
| 12 | 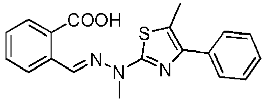 | 12-1 | 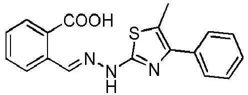 |
| 16 | 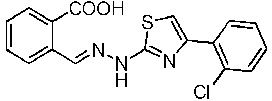 | 23 | 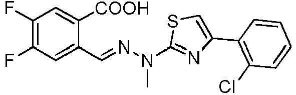 |
| 23-1 | 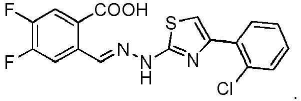 | | |

In a preferred embodiment, in the compound of formula II,
R₁ is selected from: H, a C1-C3 alkyl;
R₃ is selected from: H, a substituted or unsubstituted C1-C3 alkyl;
R₅ and R₆ are independently selected from: H, a unsubstituted or halogen-substituted C1-C3 alkyl;
R₇ and R₈ are independently selected from: H, Cl (preferably, R₇ is Cl, R₈ is H);
R₉ is H.

In a preferred embodiment, the compound is selected from compound No. 1 or No. 16.

In the second aspect, the present invention provides a pharmaceutical composition comprising a combination of a compound of the first aspect, or a pharmaceutically acceptable salt thereof and other antiviral drugs, and pharmaceutically acceptable carriers or excipients.

In a preferred embodiment, the pharmaceutical composition is used against RNA virus infection.

In a preferred embodiment, the RNA viruses include, but not limited to, coronaviruses, such as severe acute respiratory syndrome coronavirus (SARS-CoV), Middle East Respiratory Syndrome Coronavirus (MERSCoV) and 2019 novel coronavirus (2019-nCoV); Ebola virus, bunya virus, avian influenza H9N2, H1N1, H7N9, arena virus, rabies virus, hepatitis C HCV, hepatitis B HBV, human immunodeficiency virus HIV-1, herpes simplex virus HSV-1, HSV-2, etc.

In a preferred embodiment, the RNA viruses include, but not limited to: severe acute respiratory syndrome coronavirus (SARS-CoV), Middle East Respiratory Syndrome Coronavirus (MERS-CoV) and 2019 novel coronavirus (2019-nCoV) , Ebola virus, fever with thrombocytopenia syndrome virus (sftsv), avian influenza virus H9N2, H1N1.

In a preferred embodiment, the other antiviral drugs are selected from one or more of lopinavir, ritonavir, ribavirin, remdesivir, oseltamivir, Tamiflu, lanimil, Wepalamivir, Arbidol and Chloroquine (Chloroquine Phosphate), etc.; preferably one or more of oseltamivir, lopinavir, ritonavir, ribavirin, remdesivir and chloroquine (chloroquine phosphate).

In a third aspect, the present invention provides the use of a pharmaceutical composition in the preparation of a medicament for treating RNA virus infection, wherein the pharmaceutical composition contains the compound described in the first aspect or a pharmaceutically acceptable salt thereof, and pharmaceutically acceptable carriers or excipients.

In a preferred embodiment, the RNA viruses include, but not limited to, coronaviruses, such as severe acute respiratory syndrome coronavirus (SARS-CoV), Middle East Respiratory Syndrome Coronavirus (MERSCoV) and 2019 novel coronavirus (2019-nCoV); Ebola virus, bunya virus, avian influenza H9N2, H1N1, H7N9, arena virus, rabies virus, hepatitis C HCV, hepatitis B HBV, human immunodeficiency virus HIV-1, herpes simplex virus HSV-1, HSV-2, etc.

In a preferred embodiment, the RNA viruses include, but not limited to: severe acute respiratory syndrome coronavirus (SARS-CoV), Middle East Respiratory Syndrome Coronavirus (MERS-CoV) and 2019 novel coronavirus (2019-nCoV) , Ebola virus, fever with thrombocytopenia syndrome virus (sftsv), avian influenza virus H9N2, H1N1.

In a fourth aspect, the present invention provides a pharmaceutical composition for treating RNA virus infection and comprising the compound of the first aspect or a pharmaceutically acceptable salt thereof, and a pharmaceutically acceptable carrier or excipient.

In a preferred embodiment, the RNA viruses include, but not limited to, coronaviruses, such as severe acute respiratory syndrome coronavirus (SARS-CoV), Middle East Respiratory Syndrome Coronavirus (MERSCoV) and 2019 novel coronavirus (2019-nCoV); Ebola virus, bunya virus, avian influenza H9N2, H1N1, H7N9, arena virus, rabies virus, hepatitis C HCV, hepatitis B HBV, human immunodeficiency virus HIV-1, herpes simplex virus HSV-1, HSV-2, etc.

In a preferred embodiment, the RNA viruses include, but not limited to: severe acute respiratory syndrome coronavirus (SARS-CoV), Middle East Respiratory Syndrome Coronavirus (MERS-CoV) and 2019 novel coronavirus (2019-nCoV) , Ebola virus, fever with thrombocytopenia syndrome virus (sftsv), avian influenza virus H9N2, H1N1.

In the fifth aspect, the present invention provides a method for treating RNA virus infection, comprising administering a therapeutically effective amount of the compound described in the first aspect or the pharmaceutical composition described in the second aspect to a subject in need of the treatment for viral infections.

In a preferred embodiment, the RNA viruses include, but not limited to, coronaviruses, such as severe acute respiratory syndrome coronavirus (SARS-CoV), Middle East Respiratory Syndrome Coronavirus (MERSCoV) and 2019 novel coronavirus (2019-nCoV); Ebola virus, bunya virus, avian influenza H9N2, H1N1, H7N9, arena virus, rabies virus, hepatitis C HCV, hepatitis B HBV, human immunodeficiency virus HIV-1, herpes simplex virus HSV-1, HSV-2, etc.

In a preferred embodiment, the RNA viruses include, but not limited to: severe acute respiratory syndrome coronavirus (SARS-CoV), Middle East Respiratory Syndrome Coronavirus (MERS-CoV) and 2019 novel coronavirus (2019-nCoV) , Ebola virus, fever with thrombocytopenia syndrome virus (sftsv), avian influenza virus H9N2, H1N1.

It should be understood that, within the scope of the present invention, each technical feature of the present invention described above and in the following (as examples) may be combined with each other to form a new or preferred technical solution, which is not listed herein due to the limitation on contents.

### Description of drawings

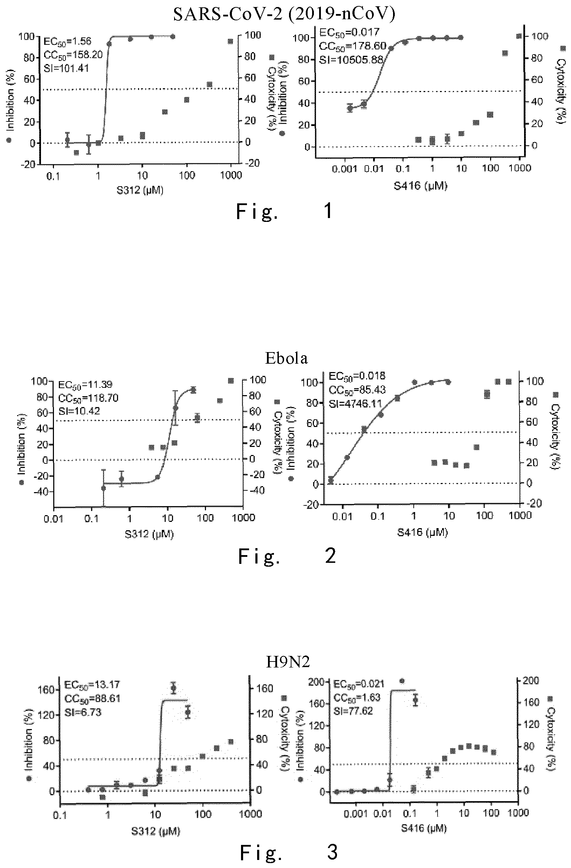

Figure 1 shows the inhibitory efficiency of compound No. 16 (S312) and compound No. 1 (S416) on 2019-nCoV on Vero cells. In the figures, the abscissa is the drug concentration, the ordinate is the inhibitory efficiency on the new coronavirus, and the number of viral gene copies in the cell supernatant is used as the quantitative standard. The red line is the virus inhibition-concentration curve, and the green line is the cytotoxicity-concentration curve;
Figure 2 shows the inhibitory efficiency of compounds S312 and S416 on Ebola replicon on BSR T7/5 cells. In the figures, the abscissa is the drug concentration, the ordinate is the inhibitory efficiency on Ebola virus replication, and the expression level of the reporter gene on the virus genome is used as a quantitative index. The red line is the virus inhibition-concentration curve, and the green line is the cytotoxicity-concentration curve;
Figure 3 shows the inhibitory efficiency of compounds S312 and S416 on H9N2 virus on MDCK cells. In the figures, the abscissa is the drug concentration, the ordinate is the inhibitory efficiency on influenza virus replication, and the CPE degree of cell death caused by virus infection (quantitatively determined by Cell Titer-Glo) is used as a quantitative index. The red line is the virus inhibition-concentration curve, and the green line is the cytotoxicity-concentration curve.

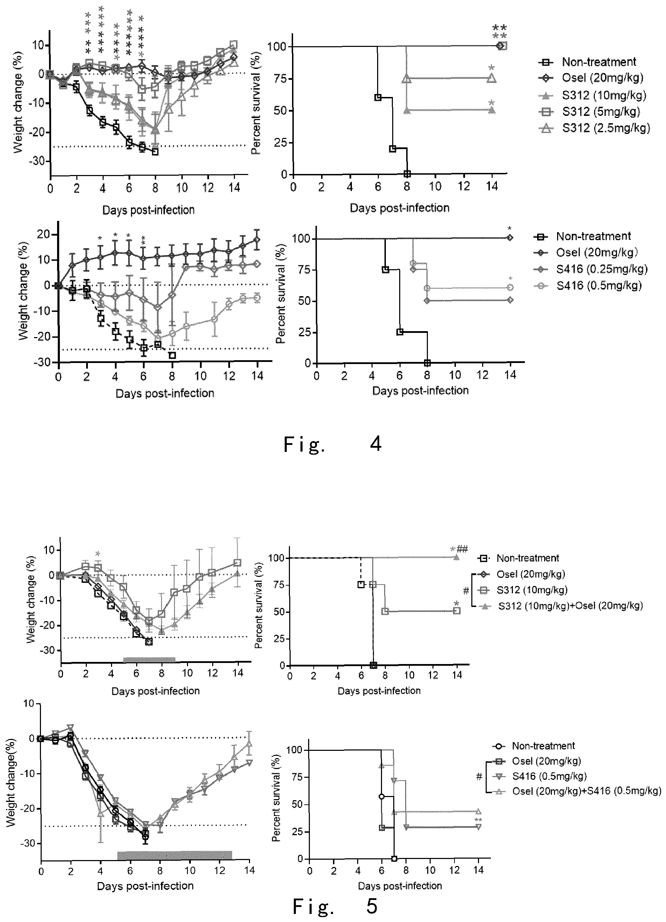

Figure 4 shows the antiviral efficacy of compounds S312 and S416 administered in the early stage of A/WSN/33(H1N1)(WSN) influenza virus infection mouse model. In the figures, the abscissa of the body weight curve on the left panel is the infection time, and the ordinate is the average percentage of body weight change ± SEM. The abscissa of the survival curve on the right panel is the infection time, and the ordinate is the survival percentage. The black line represents the Non-treatment group, the blue line represents the marketed control drug Oseltamivir (Osel)-20 mg/kg group, and the green, red and orange lines represent the test drugs. Asterisks (^{∗}) indicate significance when the administration group is compared with the Non-treatment group. ^{∗}P<0.05; ^{∗∗}P<0.01; and ^{∗∗∗}P<0.001.
Figure 5 shows the antiviral efficacy of compounds S312 and S416 administered in the intermediate and late stage of WSN influenza virus infection mouse model. In the figures, the abscissa of the body weight curve on the left panel is the infection time, the green line on the abscissa represents the administration time, and the ordinate is the mean percentage of body weight change ± SEM. The abscissa of the survival curve on the right panel is the infection time, and the ordinate is the survival percentage. The black line represents the Non-treatment group, the blue line represents the oseltamivir-20 mg/kg group, the red line represents the test compound alone group, and the green line represents the group of the test compound in combination with oseltamivir. When the drug group is compared with the Non-treatment group, an asterisk (^{∗}) indicates significance, and a pound sign (#) indicates significance between single treatment and combination treatment. ^{∗}#P<0.05; ^{∗∗}##P<0.01; and ^{∗∗∗}P<0.001.

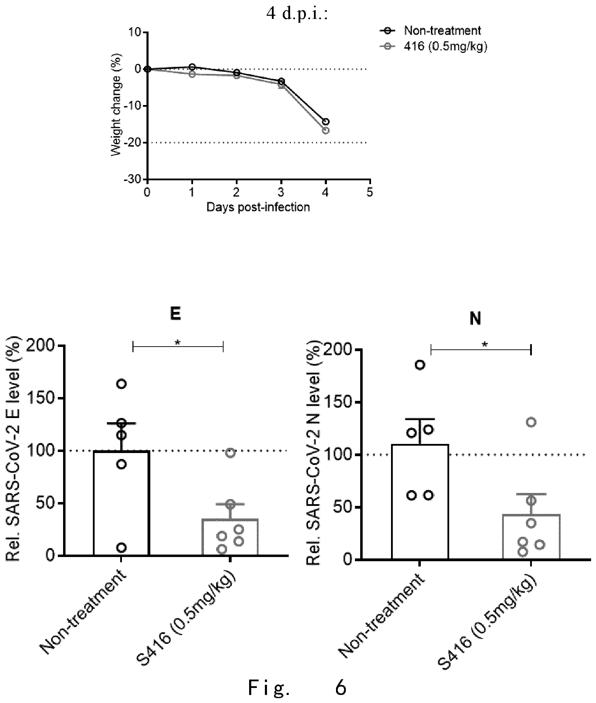

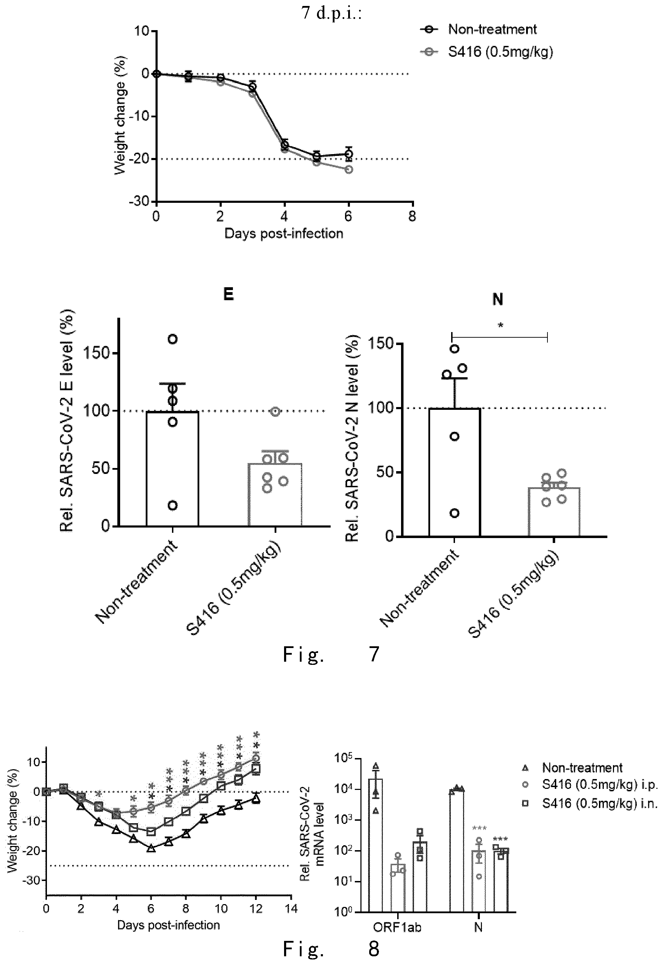

Figure 6 (Day 4 after infection) and Figure 7 (Day 7 after infection) show the antiviral efficacy of compound S416 in a mouse model of SARS-CoV-2 infection. In the figures, the abscissa of the body weight curve on the upper panel is the infection time, and the ordinate is the average percentage of body weight change ± SEM. The abscissa on the lower panels is the viral gene, and the ordinate is Relative fluorescence quantification of mRNA levels of SARS-CoV-2 genes (E gene and N gene) relative to the Non-treatment group as a percentage ± SEM. The black line represents the Non-treatment group and the red line represents the compound group. Asterisks (^{∗}) indicate significance when the compound group is compared with the Non-treatment group. ^{∗}P<0.05; ^{∗∗}P<0.01; and ^{∗∗∗}P<0.001.
Figure 8 shows the antiviral efficacy of compound S416 in a hamster model of SARS-CoV-2 infection. In the figures, the abscissa of the body weight curve on the left panel is the infection time, and the ordinate is the average percentage of body weight change ± SEM. The abscissa on the right panel is the viral gene, and the ordinate is the relative fluorescence quantification of the mRNA levels of two SARS-CoV-2 genes (ORFlab and N). The black line represents the Non-treatment group, and the red and blue lines represent the compound group. Asterisks (^{∗}) indicate significance when the compound group is compared with the Non-treatment group. ^{∗}*P*<0.05; ^{∗∗}*P*<0.01; and ^{∗∗∗}*P*<0.001.

### Modes for carrying out the invention

After extensive and in-depth research, the inventor unexpectedly discovered a series of thiazole derivatives with broad-spectrum and excellent anti-coronavirus activity. These compounds exhibit lower toxicities. The present invention has been completed based on such findings.

The inventors of the present invention designed novel compounds for the nucleic acid synthesis reaction of host cells, and obtained best candidate compounds by screening the toxicity and function of the compounds. Viral replication is heavily dependent on the nucleic acid resources of host cells, and the excessive inflammatory response caused by viral infection also depends on gene expression, therefore, blocking the nucleic acid synthesis of host cells can inhibit virus replication on the one hand, and inhibit excessive inflammatory response on the other hand. In normal cells, since gene synthesis and expression are in a certain steady state and do not rely too much on new nucleic acid synthesis, the compounds of the present invention are less toxic to normal cells, but have more significant effects on virus-infected cells.

### Definition on terms

Some of the groups involved herein are defined as follows:
As used herein, "alkyl" refers to a saturated branched or straight-chain alkyl having 1-10 carbon atoms in length, and preferred alkyls include alkyls having 2-8 carbon atoms, 1-6, 1- 4 carbon atoms, 1 to 3 carbon atoms in length. Examples of alkyl include, but not limited to, methyl, ethyl, n-propyl, isopropyl, n-butyl, isobutyl, heptyl, and the like. The alkyl may be substituted with one or more substituents, such as a halogen or haloalkyl. For example, the alkyl may be an alkyl substituted with 1-4 fluorine atoms, such as trifluoromethyl, or the alkyl group may be an alkyl substituted with a fluoroalkyl.

As used herein, "cycloalkyl" refers to a saturated alkyl containing an alicyclic structure, e.g., a C3-C6 cycloalkyl. In a specific embodiment, the cycloalkyl includes, but not limited to, a cyclopropyl, cyclobutyl, cyclopentyl, or cyclohexyl. The cycloalkyl described herein may be substituted or unsubstituted, including but not limited to, substituted with one or more halogen atoms, such as fluorine atoms.

As used herein, "amino" refers to a group of formula "NRxRy", wherein Rx and Ry can be independently selected from H or a C1-C3 alkyl or C1-C3 haloalkyl. In a specific embodiment, "amino" as used herein refers to NH2.

As used herein, "halogen" refers to fluorine, chlorine, bromine and iodine. In a preferred embodiment, the halogen is chlorine or fluorine; more preferably fluorine.

### Compounds of the invention

The inventors unexpectedly discovered a series of thiazole derivatives with broad-spectrum and excellent anti-coronavirus activities. These compounds showed excellent anti-coronavirus activities in animal experiments.

In a specific embodiment, the comppund of the present invention is a compound of formula I, or a pharmaceutically acceptable salt thereof: 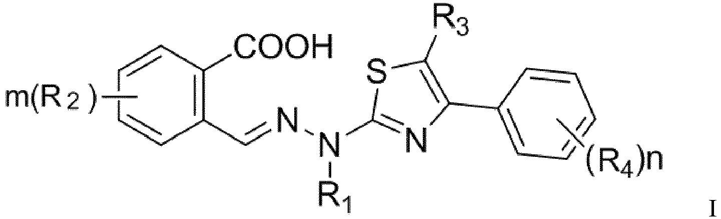 wherein,
R₁ is selected from: H, a substituted or unsubstituted C1-C6 alkyl, C3-C6 cycloalkyl;
R₂ is independently selected from: H, a halogen, substituted or unsubstituted C1-C6 alkyl, substituted or unsubstituted C1-C6 alkoxy, CN, NO₂, hydroxyl, NR^{a}R^{b};
R^{a} and R^{b} can be independently selected from H or a C1-C6 alkyl;
R₃ is selected from: H, a substituted or unsubstituted C1-C6 alkyl;
R₄ is independently selected from: H, a halogen;
m is an integer from 0 to 4;
n is an integer from 0 to 5.

In a preferred embodiment, the compound of the present invention is shown in formula II: 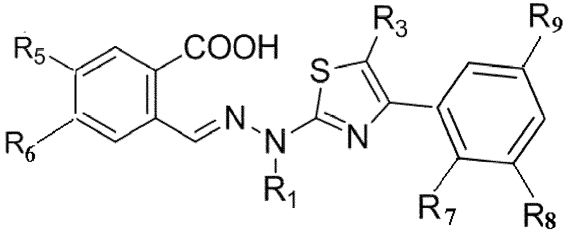 wherein,
R₅ and R₆ are independently selected from: H, a halogen, substituted or unsubstituted C1-C6 alkyl, substituted or unsubstituted C1-C6 alkoxy, hydroxyl, NH₂;
R₇, R₈ and R₉ are independently selected from: H, a halogen;
R₁ and R₃ are described as above.

In a further embodiment, R₁ is selected from: H, a C1-C6 alkyl, preferably a C1-C3 alkyl; R₃ is selected from: H, a substituted or unsubstituted C1-C6 alkyl, preferably a C1-C3 alkyl; R₅ and R₆ are independently selected from: H, a halogen, preferably F, unsubstituted or halogen-substituted (preferably F-substituted) C1-C3 alkyl; R₇ and R₈ are independently selected from: H, a halogen, preferably Cl; and R₉ is H.

In a preferred embodiment, the compound of the present invention is selected from compound No. 1 or No. 16:

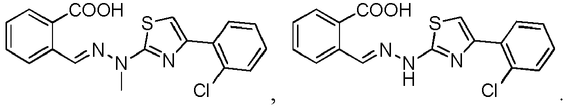

In addition, based on the common knowledge in the art and the contents of the present invention, a skilled person can know that the compounds of the present invention can form salts or esters due to the carboxyl contained therein, and can further form prodrugs.

### Virus

The RNA virus described herein is a kind of biological virus, and their genetic material is composed of ribonucleic acid (RNA). Usually the nucleic acid is single-stranded (ssRNA single-stranded RNA), but also double-stranded ( dsRNA double-stranded RNA).

The term "coronaviruses" as used herein are single-stranded positive-stranded RNA viruses belonging to Orthocoronavirinae of Coronaviridae of Nidovirales. The virus can infect humans, bats, pigs, mice, cattle, horses, goats, monkeys and many other species. Seven coronaviruses (HCoVs) are known to infect humans, including Middle East respiratory syndrome-related coronavirus (MERSr-CoV) and severe acute respiratory syndrome-related coronavirus (SARSr-CoV).

The coronavirus isolated from the lower respiratory tract of patients with pneumonia of unknown cause in Wuhan is a new type of β-type coronavirus, named by WHO as 2019-nCoV, which is the 7^{th} coronavirus that can infect humans. At present, there are no effective vaccines and therapeutic drugs against the coronavirus, but mainly preventive measures to control the spread of the virus, closely monitor the epidemic situation, and isolate suspected cases for observation. At present, there is no effective treatment for coronavirus, and symptomatic and supportive treatment is mainly adopted.

Based on the above compounds, the present invention further provides a pharmaceutical composition for treating infections caused by viruses, especially RNA viruses, including but not limited to: coronaviruses, such as severe acute respiratory syndrome coronavirus (SARS-CoV), Middle East Respiratory Syndrome Coronavirus (MERSCoV) and 2019-nCoV, Ebola Virus, Bunya Virus, Avian Influenza H9N2, H1N1, H7N9, Arenavirus, Rabies Virus, Hepatitis C HCV, Type B Hepatitis HBV, human immunodeficiency virus HIV-1, herpes simplex virus HSV-1, HSV-2 and the like. The composition contains a therapeutically effective amount of a compound of the present invention, or a pharmaceutically acceptable salt thereof, and a pharmaceutically acceptable carrier or excipient. In a preferred embodiment, the compounds or pharmaceutical compositions of the present invention can be used for treating infections caused by severe acute respiratory syndrome coronavirus (SARS-CoV), Middle East respiratory syndrome coronavirus (MERS-CoV) and 2019-nCoV, Ebola virus, fever with thrombocytopenia syndrome virus (sftsv), avian influenza virus H9N2.

Examples of pharmaceutically acceptable salts of the compounds of the present invention include, but not limited to, inorganic and organic acid salts, such as hydrochloride, hydrobromide, sulfate, citrate, lactate, tartrate, maleate, fumarate, mandelate, and oxalate; and inorganic and organic base salts with bases such as sodium hydroxy, tris(hydroxymethyl)aminomethane (TRIS, tromethamine) and N-methylglucamine.

Although each individual's needs vary, a skilled person can determine the optimal dosage of each active ingredient in the pharmaceutical compositions of the present invention. In general, a compound of the present invention, or a pharmaceutically acceptable salt thereof, is administered orally to mammals daily in an amount of about 0.0025 to 50 mg/kg body weight, preferably about 0.01 to 10 mg per kg. For example, a unit oral dosage may contain about 0.01 to 50 mg, preferably about 0.1 to 10 mg, of a compound of the present invention. A unit dosage may be administered one or more times, in one or more tablets per day, each tablet containing about 0.1 to 50 mg, preferably about 0.25 to 10 mg, of a compound of the present invention or a solvate thereof.

The pharmaceutical compositions of the present invention can be formulated into formulations suitable for various routes of administration, including but not limited to being formulated into a form for parenteral, subcutaneous, intravenous, intramuscular, intraperitoneal, transdermal, buccal, intrathecal, intracranial, nasal or topical administration for treating tumors and other diseases. The amount administered is an amount effective to ameliorate or eliminate one or more conditions. For treating a particular disease, an effective amount is an amount sufficient to ameliorate or in some way alleviate symptoms associated with the disease. Such amount can be administered as a single dose, or can be administered according to an effective therapeutic regimen. The amount administered may cure the disease, however the administration is usually to improve the symptoms of the disease. Repeated administration is generally required to achieve the desired symptomatic improvement. The dosage will be determined based on the patient's age, health and weight, the type of concurrent therapy, the frequency of therapy, and desired therapeutic benefits.

The pharmaceutical formulations of the present invention can be administered to any mammal so long as they can obtain the therapeutic effects of the compounds of the present invention. The most important of these mammals are humans. The compounds of the present invention or pharmaceutical compositions thereof can be used for treating ulcerative colitis.

The pharmaceutical formulations of the present invention can be manufactured in a known manner, for example, by conventional mixing, granulating, tableting, dissolving, or freeze-drying processes. In the manufacture of oral dosage forms, solid excipients and active compounds can be combined and the mixture can be optionally ground. After suitable auxiliaries, if desired or necessary, are added, the mixture of granules is processed to obtain tablets or dragee cores.

Suitable auxiliaries are especially fillers, for example sugars such as lactose or sucrose, mannitol or sorbitol; cellulose preparations or calcium phosphates, such as tricalcium phosphate or dicalcium phosphate; and binders, such as starch pastes, including corn starch , wheat starch, rice starch, potato starch, gelatin, tragacanth, methylcellulose, hydroxypropylmethylcellulose, sodium carboxymethylcellulose, or polyvinylpyrrolidone. If desired, disintegrants, such as the starches mentioned above, as well as carboxymethyl starch, cross-linked polyvinylpyrrolidone, agar, or alginic acid or a salt thereof, such as sodium alginate, can be added. Adjuvants are especially flow conditioners and lubricants, for example, silica, talc, stearates, such as magnesium calcium stearate, stearic acid or polyethylene glycols. If desired, dragee cores can be provided with a suitable coating resistant to gastric juices. For this purpose, concentrated sugar solutions can be used. Such solution may contain gum arabic, talc, polyvinylpyrrolidone, polyethylene glycol and/or titanium dioxide, lacquer solutions and suitable organic solvents or solvent mixtures. For preparing coatings resistant to gastric juices, suitable cellulose solutions such as cellulose acetate phthalate or hydroxypropyl methylcellulose phthalate can be used. Dyestuffs or pigments may be added to the coatings of tablets or dragee cores for, for example, the identification or characterization of combinations of active ingredients.

The administration method of the pharmaceutical composition includes but not limited to various administration methods well known in the art, and can be determined according to the actual situation of a patient. These methods include, but not limited to, parenteral, subcutaneous, intravenous, intramuscular, intraperitoneal, transdermal, buccal, intrathecal, intracranial, nasal, or topical routes of administration.

In addition to the compound of the present invention, the pharmaceutical composition of the present invention may also contain other antiviral drugs, and the other antiviral drugs may be selected from one or more of lopinavir, ritonavir, ribavirin, remdesivir, oseltamivir, Tamiflu, lanimivir, peramivir and Chloroquine (Chloroquine Phosphate), etc.; and preferably, one or more of lopinavir, ritonavir, ribavirin, remdesivir, and chloroquine (chloroquine phosphate). In a preferred embodiment, the compound that can be used in combination with other antiviral drugs in the pharmaceutical composition of the present invention is Compound No. 1 or Compound No. 16.

### Advantages of the invention

1. The present invention discovers for the first time a series of thiazole derivatives with broad-spectrum and excellent anti-RNA virus, especially coronavirus activities;
2. The compounds of the present invention have low toxicities to normal cells; and
3. The compounds of the present invention have laid a material foundation for the research and development of a new generation of anti-RNA viruses, especially anti-coronavirus drugs, and thus have very important academic value and practical significance.

The present invention will be further described below with reference to specific examples. These examples are not intended to limit the scope of the invention. All the methods adopted according to the principles and technical means of the present invention belong to the scope of the present invention. In the following examples, the experiment method without specifying the specific conditions is usually based on the conventional conditions or the conditions recommended by the manufacturer. Percentages and parts are percentages and parts by weight, unless otherwise indicated.

### Example 1: Reference for the preparation of compounds

With reference to CN107459496A, the following compounds were prepared.

| No. | Structure of compound | No. | Structure of compound |
|---|---|---|---|
| 1(S416) | 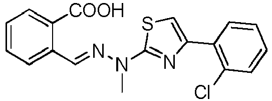 | 2 | 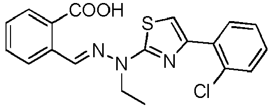 |
| 3 | 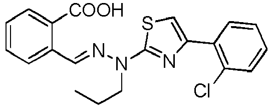 | 4 | 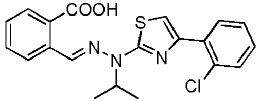 |
| 5 | 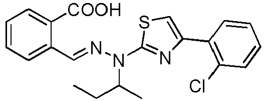 | 6 | 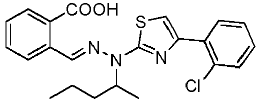 |
| 7 | 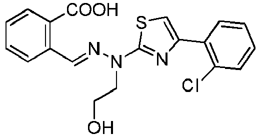 | 8 | 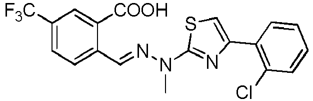 |
| 8-1 | 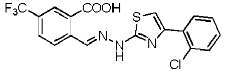 | 9 | 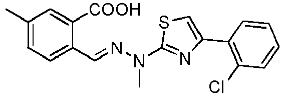 |
| 9-1 | 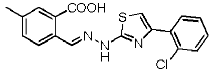 | 10 | 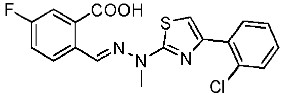 |
| 10-1 | 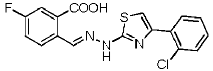 | 11 | 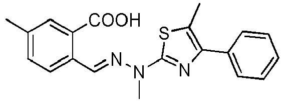 |
| 12 | 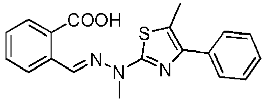 | 12-1 | 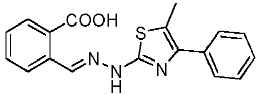 |
| 13 | 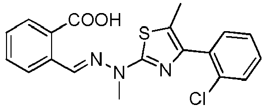 | 14 | 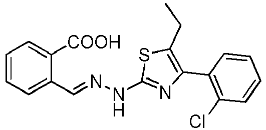 |
| 15 | 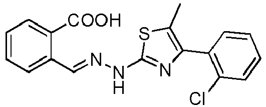 | 16(S312) | 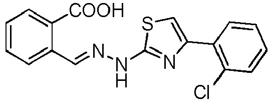 |
| 17 | 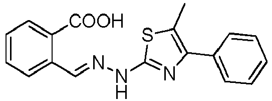 | 18 | 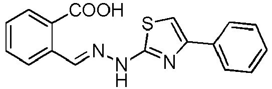 |
| 19 | 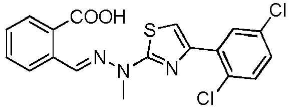 | 20 | 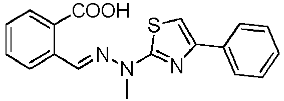 |
| 21 | 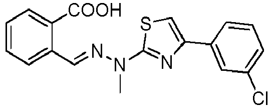 | 22 | 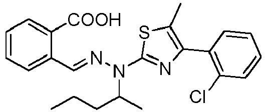 |
| 23 | 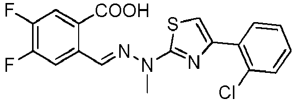 | 23-1 | 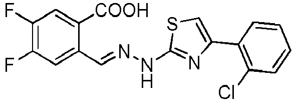 |
| 24 | 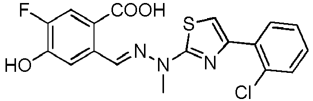 | 25 | 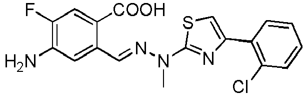 |
| 52 | 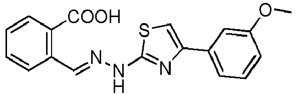 | 53 | 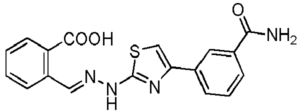 |
| 54 | 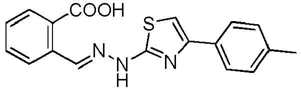 | 55 | 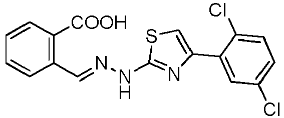 |
| 56 | 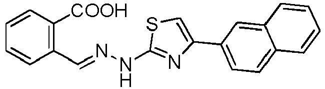 | 57 | 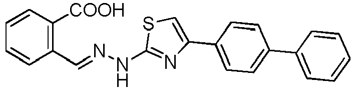 |
| 58 | 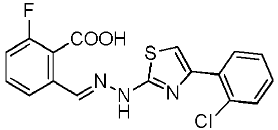 | 59 | 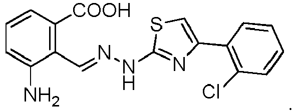 |

### Example 2. Activity evaluation

Inhibitory activity and cytotoxicity evaluation of the compounds of the present invention on 2019 novel coronavirus (2019-nCoV), Ebola virus, avian influenza virus A/GuangZhou/99 (H9N2)
Materials and Methods: Compound 16 (S312) and Compound 1 (S416)

### Detection methods and results:

### 1. Experiment using fluorescence quantitative PCR method to detect the efficacy of anti-2019-nCoV:

Vero E6 cells (ATCC-1586) were infected with 2019BetaCoV/Wuhan/WIV04/2019 strain (isolated by Wuhan Institute of Virology, Chinese Academy of Sciences) at a MOI = 0.05, and at the same time, different dilution concentrations of drugs were added for co-culture. The drugs were diluted with DMSO and mixed with DMSO dilution served as a control. The infection solution was DMEM + 0.2% BSA. After 48 hours, the cell supernatant was collected, and the viral RNA in the supernatant was extracted by a viral RNA extraction kit (Qiagen), and the number of copies of viral RNA in the cell supernatant was detected by real-time quantitative reverse transcription PCR (qRT-PCR) (Qiagen) to reflect the replication efficiency of the virus. Data processing was performed by Graphpad prism software, and the half inhibitory concentration (IC₅₀) of the compound against virus was calculated.

### 2. Experiment to test the efficacy of anti-Ebola virus replicon by Bright-Glo

### Ebola virus replicon system (EBOV-NP, EBOV-VP35, EBOV-VP30, EBOV-MG, EBOV-L)

From the list of human-to-human pathogenic microorganisms, it can be seen that the hazard degree of Ebola virus is classified as the first category, which must be operated in a laboratory with a BSL-4 biosafety level. In order to reduce the risk of biosafety, Ebola virus replicon system was used to test the antiviral efficacy. This system can completely reflect the efficiency of Ebola virus replication and is a commonly used system for screening anti-Ebola virus drugs. The Ebola virus replicon system consists of a mini-genome expression plasmid MG recombinantly expressing T7 promoter and the luciferase gene, and 4 helper plasmids that express L, NP, VP35, and VP30 proteins, respectively. When the replicon system is transfected into a cell for replication, T7 RNA polymerase induces the initiation of T7 promoter, which in turn drives the expression of the luciferase gene. The replicon replication efficiency is positively correlated with the luciferase gene expression, therefore, the luciferase gene expression in the drug-treated cell system can be used to measure the replicon replication efficiency in the evaluation of a drug, so that the inhibition degree of the drug on Ebola replicon can be evaluated. Bright-Glo reagent (Promega) was used for the detection of luciferase expression. 2×10⁴ of BSR T7/5 cells (which was stably transfected with a gene expressing T7 RNA polymerase, and the cell culture medium was MEM+10% FBS+1% L-Glutamine+2% MAA+1% P/S) were plated in a 96-well plate with a white bottom and used when the cells reached 80% confluence. The five-plasmid replication system for Ebola virus was prepared as follows:

| System | Concentrat ion (ng) |
|---|---|
| EBOV-NP | 25 |
| EBOV-VP35 | 25 |
| EBOV-VP30 | 15 |
| EBOV-MG | 25 |
| EBOV-L | 150 |
| Total concentration | 240 |

Transfection: the plasmids of the above system were dissolved in 25 µL of Opti-MEM serum-free medium, marked as tube A; and 0.72 µL of Lipo 2000 was dissolved in 25 µL of Opti-MEM serum-free medium, marked as tube B. Tubes A and B were mixed and recorded as tube C, and placed at room temperature for 20 min. Compounds were added to the treated 96-well plate at 50 µL/well (the system without EBOV-L plasmid was used as the negative control). The plate was shaken at 800 rpm for 2 hours, the compounds were 3-fold diluted with Opti-MEM serum-free medium at 8 gradients, in triplicate, added to a shaken 96-well plate with white bottom at 50 µL per well, and placed in a 37°C, 5 %CO₂ incubator for 24 hours. And then, 50 µL of Bright-Glo reagent was added to each well, shaken for 3 min in the darkness, mixed well, and kept stand for 10 min. The plate was placed on a microplate reader for recording the Luminescence value, and the data were processed by Graphpad prism software to obtain the half inhibitory concentration of the compound on the virus IC₅₀.

### 3. Experiment for Detecting Efficacy Against Human Infectious Avian Influenza Virus by Cell Titer-Glo

This experiment was based on the luminescence detection method of Cell Titer-Glo reagent, and the inhibitory activities of the compounds against the avian influenza virus A/GuangZhou/99 (H9N2) (the virus was donated by the National Influenza Center), which can infect humans, were detected. 2 × 10⁴ of MDCK cells were plated in a 96-well cell culture plate with a white bottom. After the cells grew into a monolayer, the original culture medium was discarded, and 50 µL of 20 TCID50 H9N2 avian influenza virus suspension and 50 µL of drug diluted solution were added at the same time, at least triplicate wells for each concentration. The virus infection solution is DMEM + 0.2% BSA + 25 mM HEPES + 1 µg/mL TPCK. A normal cell control group and a virus control group were also set. The 96-well cell culture plate was placed in a 37°C, 5% CO₂ incubator, and the CPE caused by the virus was observed under a microscope every day. The plate was removed from the incubator when 75%-100% CPE appeared in the virus control group. 25 µL of CellTiter-Glo^{®} reagent was added to each well, shaken for 3 min in the darkness, mixed well, and kept stand for 10 min. The plate was placed on a microplate reader for recording the Luminescence value, and the data were processed by Graphpad prism software to obtain the half inhibitory concentration of the compound on the virus IC₅₀.

### 4. Experiment to detect drug toxicity by CellTiter-Glo (CC₅₀)

Adenosine Tri-Phosphate (ATP) is involved in a variety of enzymatic reactions in organisms and is an indicator of the metabolism of living cells. The survival of cells can be detected by detecting the content of ATP in cells. In CellTiter-Glo live cell detection, luciferase is used as the detection substance. During the luminescence process, luciferase requires the participation of ATP. ATP can only be generated through the respiration of metabolically active cells and other life activities. An equal volume of CellTiter-Glo reagent was added to the cell culture medium, and the luminescence value was measured. The light signal is proportional to the amount of ATP in the system, and ATP is positively correlated with the number of living cells, so that the survival of the cells can be calculated. 2×10⁴ of cells were plated in a 96-well plate with a white bottom. After the cells grew into a monolayer, the original culture medium was discarded, and the compounds were diluted with infection medium (DMEM+0.2%BSA+25mM HEPES+1µg/mL TPCK) to different concentrations and added to a 96-well plate with 100 µl per well, 5 replicate wells for each concentration. The cells, into which only 0.1 mL of infection solution was added, were the normal control group. The plate was placed in a 37°C, 5% CO₂ incubator for 72 hours, afterwards taken out of the incubator and cooled to room temperature. 25 µL of CellTiter-Glo reagent was added to each well, shaken for 3 min in the darkness, mixed well, and kept stand for 10 min. The plate was placed on a microplate reader for recording the Luminescence value, and the data were processed by Graphpad prism software to obtain the semitoxic concentration (CC₅₀) and nontoxic limit concentration (MNCC) of compounds to cells. Cell viability % = Luminescence value of test well / Luminescence value of cell control well × 100%.

### 5. Determination of in vivo activities of compounds anti-H1N1 virus and 2019-nCoV

### 5.1 Test for Efficacy of compounds S312 and S416 Against A/WSN/33(H1N1) Virus

In this experiment, the anti-influenza efficacy of the drug was evaluated based on a mouse influenza virus infection model. The mice used were BALB/c female mice, 6-8 weeks old, weighing 18-22 g, and purchased from Beijing Weitong Lihua Laboratory Animal Technology Co., Ltd. All animal experiments were performed in the ABSL-2 laboratory. The mice were acclimated to the ABSL-2 environment for 2-3 days before the experiment. All mice were intranasally infected with A/WSN/33(H1N1)2LD₅₀ = 2000 pfu/mice. The early administration was performed 3 hours before infection, and then once a day for 14 consecutive days. Administrations during the middle and late period is the number of days of administration marked in the figure, and the administration started from the day 5 after infection, and continued for 5 or 9 days. Body weight and survival data of mice were plotted using Graphpad prism software to draw body weight change and survival rate curves, expressed as mean ± standard deviation (mean ± S.E.M.), and two-way ANOVA was used for statistical analysis. Compared with the control group, when P<0.05, it was considered to be statistically significant.

### 5.2 Anti-2019-nCoV efficacy test of compound S416 in hACE2 transgenic mouse model

The mice used in this experiment were K18-hACE male mice, 6-8 weeks old, 19-28 g, purchased from Jiangsu Jicui Yaokang Biotechnology Co., Ltd. All animal experiments were performed in the ABSL-3 laboratory. The mice were acclimated to the ABSL-3 environment for 2-3 days before the experiment, and the mice were divided into following two groups: model group: Non-treatment; experimental group: S416-0.5 mg/kg; 10-12 mice in each group. After being anesthetized, the mice were intranasally infected with 2.5 × 10² of 2019-nCoV. Mice were administered intraperitoneally 2 hours before infection, and administered continuously for 3 days (Fig. 6) or 5 days (Fig. 7), once a day; Graphpad prism software was used to draw the weight and survival curve for the mice in each group. On day 4 and 7, the mice were euthanized and the lungs were taken out. The lungs were ground and processed to extract RNA, and fluorescence quantification was performed to compare the copy numbers of virus E and N genes in the lung tissue of mice in each group.

### 5.3 Anti-2019-nCoV efficacy test of compound S416 in hamster model

The hamsters used in this experiment were Syrian hamster male rats, 6-8 weeks old, 100-120 g, and purchased from Beijing Weitong Lihua Laboratory Animal Technology Co., Ltd. All animal experiments were performed in the ABSL-3 laboratory. The hamsters were acclimated to the ABSL-3 environment for 2-3 days before the experiment. The hamsters were divided into following 3 groups: model group: Non-treatment; experimental group: 416-0.25 mg/kg intraperitoneal administration (i.p.), 416-0.5 mg/kg intranasal administration (i.n.); 6 per group. After being anesthetized, the hamsters were intranasally infected with 2019-nCoV at 1 × 10⁷ virus. Administration was performed for 5 consecutive days, once a day. Graphpad prism software was used to draw the weight and survival curve for hamsters in each group. On day 7, some hamsters were euthanized and their lungs were taken out. After the lungs were ground, RNA extraction was performed, and fluorescence quantification was performed to compare the copy numbers of virus ORFlab and N genes in the lung tissue of hamsters in each group. Body weight data of mice are presented as mean ± S.E.M., and statistical analysis was performed using two-way ANOVA. Lung virus titers were statistically analyzed using t-test. Compared with the control group, when P<0.05, it was considered to be statistically significant.

### Experimental results:

**Table 1. Summary of antiviral efficacy of compound 16 (S312) and compound 1 (S416) on 2019-nCoV, Ebola replicon, and avian influenza H9N2 infecting human**

| Virus species | EC₅₀/CC₅₀ µM (SI) | |
|---|---|---|
| | S312 | S416 |
| 2019-nCoV | 1.56/158.20 (101.41) | 0.017/178.60 (10505.88) |
| Ebola replicon | 11.39/118.7 (10.42) | 0.018/85.84 (4746.11) |
| A/GuangZhou/99(H9N2) | 13.17/88.61 (6.73) | 0.021/1.63 (77.62) |

Examples of the above assays for EC50 activity are as follows:
1. The activities of S312 and S416 on 2019-nCoV is that when the concentration of S312 is 1.55 µM and the concentration of S416 is 0.017 µM, the replication efficiency of 2019-nCoV in Vero E6 cells can be inhibited by 50%. As shown in Figure 1.
2. The inhibitory activities of S312 and S416 on Ebola replicon is that when the concentration of S312 is 14.96 µM and the concentration of S416 is 0.018 µM, the replication efficiency of the virus can be inhibited by 50%. As shown in Figure 2.
3. The inhibitory activities of S312 and S416 on avian influenza virus H9N2 is that when the concentration of S312 is 13.17 µM and the concentration of S416 is 0.020 µM, the replication efficiency of the virus can be inhibited by 50%. As shown in Figure 3.

### Example 3. Activity evaluation of a series of compounds

Inhibitory activities of the series of compounds of the present invention against 2019-nCoV, Ebola virus, and avian influenza virus A/GuangZhou/99 (H9N2) (methods were the same as above)

| No. of compound | 2019-nCoV EC₅₀ (µM) | Ebola virus EC₅₀ (µM) | A/GuangZhou/99(H9N2) EC₅₀ (µM) |
|---|---|---|---|
| 1(S416) | 0.017 | 0.018 | 0.021 |
| 2 | 0.16 | 0.13 | 0.28 |
| 3 | 2.35 | 5.42 | 2.38 |
| 4 | 10.20 | 5.35 | 8.37 |
| 5 | >100 | 89 | 45.37 |
| 6 | >100 | 97 | 22.18 |
| 7 | - | - | - |
| 8 | >100 | 49 | >100 |
| 8-1 | 5.25 | 1.14 | >100 |
| 9 | 0.29 | 2.26 | 1.37 |
| 9-1 | 0.07 | 0.89 | 0.33 |
| 10 | 0.10 | 0.32 | 3.20 |
| 10-1 | 0.11 | 0.97 | 0.33 |
| 11 | >100 | - | - |
| 12 | 0.35 | - | - |
| 12-1 | 0.52 | - | - |
| 13 | >100 | - | 8.27 |
| 14 | - | - | - |
| 15 | - | - | - |
| 16(S312) | 1.56 | 11.39 | 13.17 |
| 17 | - | - | - |
| 18 | - | - | - |
| 19 | - | - | - |
| 20 | - | - | - |
| 21 | - | - | - |
| 22 | - | - | - |
| 23-1 | 5.44 | - | - |
| 24 | - | - | - |
| 25 | - | - | - |
| 52 | - | - | - |
| 53 | - | - | - |
| 54 | - | - | - |
| 55 | 1.02 | 5.40 | 4.06 |
| 56 | - | - | - |
| 57 | - | - | - |
| 58 | - | - | - |
| 59 | - | - | - |

| | | | |
|---|---|---|---|
| "-" **indicates that the EC₅₀ value of a compound is to be detected.** | | | |

### Example 4. Evaluation of the antiviral activities of the compound of the present invention in combination with other antiviral drugs (2019-nCoV, Ebola virus, avian influenza virus A/GuangZhou/99 (H9N2)) (methods were the same as above)

The present inventors further tested comninations of the compounds of the present invention with other antiviral drugs in the prior art, including one or more of lopinavir, ritonavir, ribavirin, remdesivir, oseltamivir, Tamiflu, lanimirvir, Peramivir, and Chloroquine (Chloroquine Phosphate).

The results found that the compound 16 (S312) and compound 1 (S416) of the present invention can produce better therapeutic effects when used in combination with these antiviral drugs; and the therapeutic effects of the combination of the compounds with lopinavir, ritonavir, ribavirin, remdesivir, or chloroquine (chloroquine phosphate) are relatively good.

### Example 5. Evaluation of the anti-influenza virus efficacy of the compounds of the present invention alone or in combination with other antiviral drugs in a mouse infection model

The inventors further tested the anti-influenza virus efficacy of the compounds of the present invention in a mouse infection model. In the early stage of influenza virus infection in mice, the compounds were administered alone and in combination in the middle and late stages to verify the early and middle and late anti-influenza efficacy of the compounds.

Results showed that in the mouse model of complete lethality, the early intraperitoneal administration of S312 (2.5-10 mg/kg) could treat 50%-100% of the mice, and the early intraperitoneal administration of S416 (0.25-0.5 mg/kg) could treat 50% or higher of the mice. As shown in Figure 4.

Intraperitoneal administration of S312 (10 mg/kg) in the middle and late stages can treat 50% of the mice, and 100% of the mice can be treated in combination with oseltamivir. Intraperitoneal administration of S416 (0.5 mg/kg) in the middle and late stages can treat 28.6% of mice, and more than 42.9% of mice can be treat in combination with oseltamivir. As shown in Figure 5.

### Example 6. Pharmacological evaluation of the compounds of the present invention against 2019-nCoV in mice and hamster infection models

The inventors further tested the efficacy of the compounds of the present invention against 2019-nCoV in mouse and hamster models. The drug was administered in the early stages of infection of 2019-nCoV in mice and hamsters to verify the early efficacy of the compound against 2019-nCoV.

In a mouse complete lethal model, intraperitoneal administration of S416 at 0.5 mg/kg on day 4 after infection did not significantly improve weight loss and death caused by viral infection, but significantly reduced viral load in lung tissue by more than 56% . As shown in Figure 6. On day 7 after infection, intraperitoneal administration of 0.5 mg/kg of S416 could not significantly improve the weight loss and death caused by viral infection, but it could significantly reduce the viral load in lung tissue by more than 45%. As shown in Figure 7.

In a non-lethal model of hamster infection, the administration of S416 significantly slowed viral infection-induced weight loss and significantly reduced viral load in lung tissue by more than 100-fold. As shown in Figure 8.

### Discussion

It can be seen from the above results that the series of compounds of the present invention have high inhibitory activities against 2019-nCoV, Ebola virus, and avian influenza virus A/GuangZhou/99 (H9N2). Especially, compound 16 (S312 ), compound 1 (S416) has high activities, EC₅₀ values can reach the level of 10 nM, and has excellent safety, which have good application prospects. In view of the current 2019-nCoV epidemic around the world including China, it is necessary to speed up the research of the application of the compounds of the present invention.

All literatures mentioned in the present application are incorporated by reference herein, as though individually incorporated by reference. Additionally, it should be understood that after reading the above teaching, many variations and modifications may be made by the skilled in the art, and these equivalents also fall within the scope as defined by the appended claims.

## Claims

1. Use of a compound of formula I, or a pharmaceutically acceptable salt thereof, in the preparation of a drug against RNA virus infection: 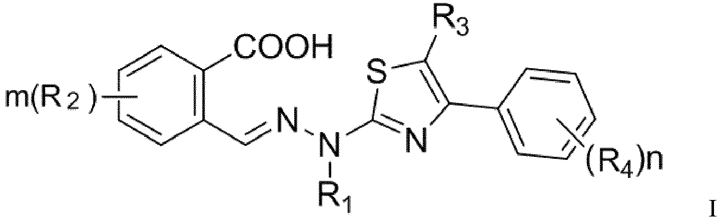 wherein,
R₁ is selected from: H, a substituted or unsubstituted C1-C6 alkyl, C3-C6 cycloalkyl;
R₂ is independently selected from: H, a halogen, substituted or unsubstituted C1-C6 alkyl, substituted or unsubstituted C1-C6 alkoxy, CN, NO₂, hydroxyl, NR^{a}R^{b};
R^{a} and R^{b} can be independently selected from H or a C1-C6 alkyl;
R₃ is selected from: H, a substituted or unsubstituted C1-C6 alkyl;
R₄ is independently selected from: H, a halogen;
m is an integer from 0 to 4;
n is an integer from 0 to 5.

2. The use of claim 1, wherein the RNA viruses include, but not limited to, coronaviruses, such as severe acute respiratory syndrome coronavirus (SARS-CoV), Middle East Respiratory Syndrome Coronavirus (MERSCoV), and 2019-nCoV; Ebola virus, bunya virus, avian influenza H9N2, H1N1, H7N9, arena virus, rabies virus, hepatitis C HCV, hepatitis B HBV, human immunodeficiency virus HIV-1, herpes simplex virus HSV-1, HSV-2, etc.

3. The use of claim 2, wherein the RNA viruses include, but not limited to: severe acute respiratory syndrome coronavirus (SARS-CoV), Middle East Respiratory Syndrome Coronavirus (MERS-CoV) and 2019 novel Coronavirus (2019-nCoV), Ebola virus, fever with thrombocytopenia syndrome virus (sftsv), avian influenza virus H9N2, H1N1.

4. The use of any one of claims 1-3, wherein the compound is shown in formula II: 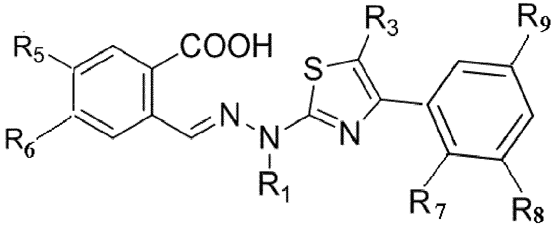 wherein,
R₅ and R₆ are independently selected from: H, a halogen, substituted or unsubstituted C1-C6 alkyl, substituted or unsubstituted C1-C6 alkoxy, hydroxyl, NH₂;
R₇, R₈ and R₉ are independently selected from: H, a halogen;
R₁ and R₃ are described as in claim 1.

5. The use of claim 4, wherein in the formula
R₁ is selected from: H, a C1-C6 alkyl;
R₃ is selected from: H, a substituted or unsubstituted C1-C6 alkyl;
R₅ and R₆ are independently selected from: H, a halogen, unsubstituted or halogen-substituted C1-C3 alkyl;
R₇ and R₈ are independently selected from: H, a halogen;
R₉ is H.

6. The use of any one of claims 1-3, wherein the compound of formula I or a pharmaceutically acceptable salt thereof is a compound selected from the following group or a pharmaceutically acceptable salt thereof:
| No. | Structure of compound | No. | Structure of compound |
|---|---|---|---|
| 1 | 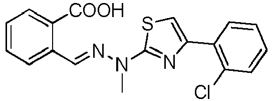 | 2 | 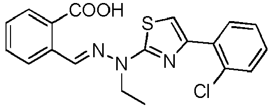 |
| 3 | 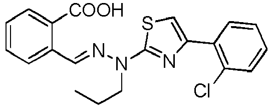 | 4 | 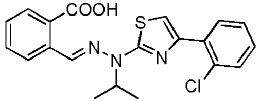 |
| 5 | 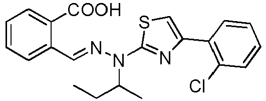 | 6 | 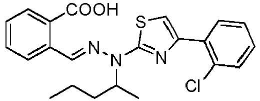 |
| 7 | 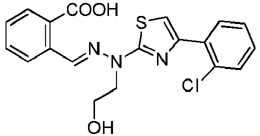 | 8 | 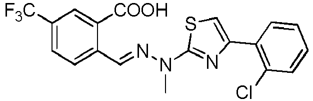 |
| 8-1 | 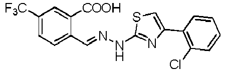 | 9 | 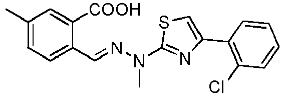 |
| 9-1 | 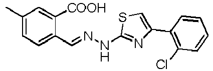 | 10 | 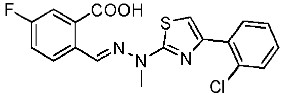 |
| 10-1 | 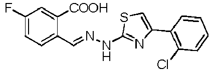 | 11 | 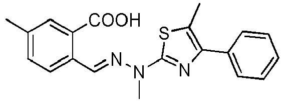 |
| 12 | 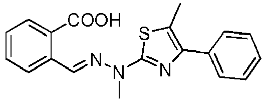 | 12-1 | 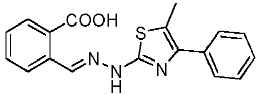 |
| 13 | 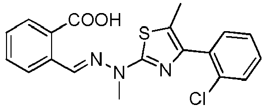 | 14 | 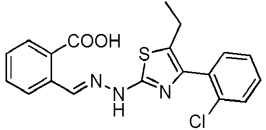 |
| 15 | 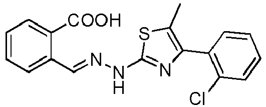 | 16 | 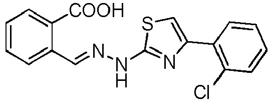 |
| 17 | 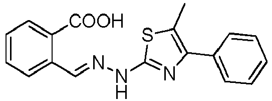 | 18 | 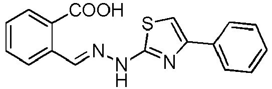 |
| 19 | 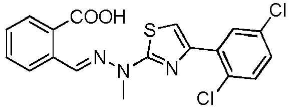 | 20 | 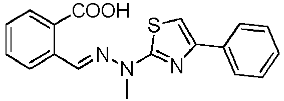 |
| 21 | 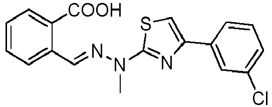 | 22 | 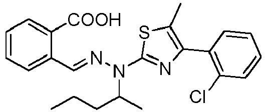 |
| 23 | 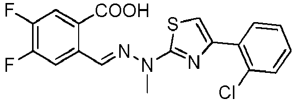 | 23-1 | 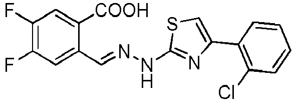 |
| 24 | 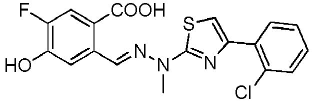 | 25 | 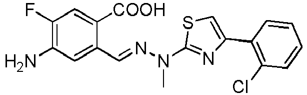 |
| 52 | 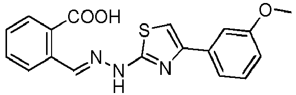 | 53 | 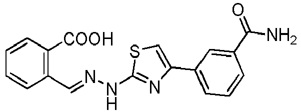 |
| 54 | 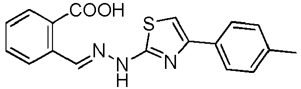 | 55 | 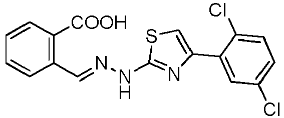 |
| 56 | 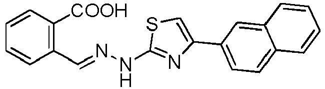 | 57 | 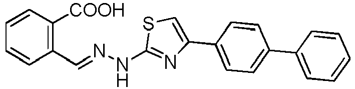 |
| 58 | 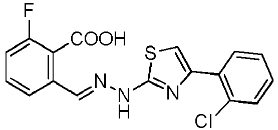 | 59 | 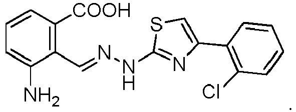 |

7. The use of claim 6, wherein the compound is a compound selected from the following group:
| No. | Structure of compound | No. | Structure of compound |
|---|---|---|---|
| 1 | 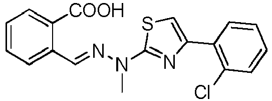 | 2 | 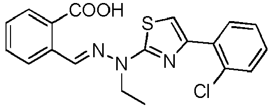 |
| 8 | 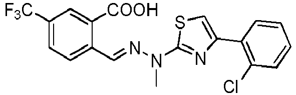 | 8-1 | 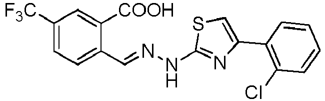 |
| 9 | 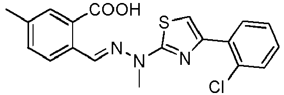 | 9-1 | 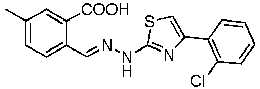 |
| 10 | 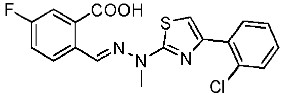 | 10-1 | 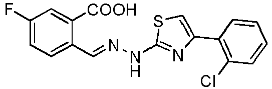 |
| 12 | 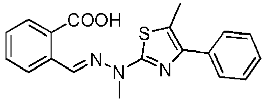 | 12-1 | 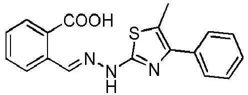 |
| 16 | 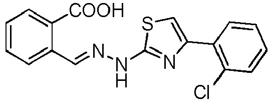 | 23 | 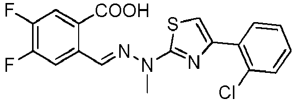 |
| 23-1 | 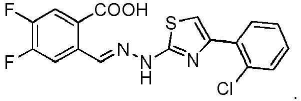 | | |

8. The use of claim 4, wherein in the compound of formula II,
R₁ is selected from: H, a C1-C3 alkyl;
R₃ is selected from: H, a substituted or unsubstituted C1-C3 alkyl;
R₅ and R₆ are independently selected from: H, a unsubstituted or halogen-substituted C1-C3 alkyl;
R₇ and R₈ are independently selected from: H, Cl (preferably, R₇ is Cl, R₈ is H);
R₉ is H.

9. The use of claim 8, wherein the compound is selected from:

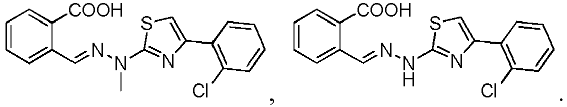

10. A pharmaceutical composition comprising a combination of a compound of any one of claims 1-9, or a pharmaceutically acceptable salt thereof and other antiviral drugs, and pharmaceutically acceptable carriers or excipients.

11. The pharmaceutical composition of claim 10, wherein the other antiviral drugs are selected from one or more of lopinavir, ritonavir, ribavirin, remdesivir, oseltamivir, Tamiflu, lanimil, Wepalamivir, Arbidol and Chloroquine (Chloroquine Phosphate), etc.; and preferably one or more of oseltamivir, lopinavir, ritonavir, ribavirin, remdesivir and chloroquine (chloroquine phosphate).

12. Use of a pharmaceutical composition in the preparation of a medicament for treating RNA virus infection, wherein the pharmaceutical composition comprises a combination of a compound of any one of claims 1-9, or a pharmaceutically acceptable salt thereof and pharmaceutically acceptable carriers or excipients.

13. The use of claim 12, wherein the RNA viruses include, but not limited to, coronaviruses, such as severe acute respiratory syndrome coronavirus (SARS-CoV), Middle East Respiratory Syndrome Coronavirus (MERSCoV) and 2019-nCoV; Ebola virus, bunya virus, avian influenza H9N2, H1N1, H7N9, arena virus, rabies virus, hepatitis C HCV, hepatitis B HBV, human immunodeficiency virus HIV-1, herpes simplex virus HSV-1, HSV-2, etc.

14. The use of claim 13, wherein the RNA viruses include, but not limited to: severe acute respiratory syndrome coronavirus (SARS-CoV), Middle East Respiratory Syndrome Coronavirus (MERS-CoV) and 2019-nCoV, Ebola virus, fever with thrombocytopenia syndrome virus (sftsv), avian influenza virus H9N2, H1N1.

15. A pharmaceutical composition for treating RNA virus infection and comprising the compound of any one of claims 1-8 or a pharmaceutically acceptable salt thereof, and a pharmaceutically acceptable carrier or excipient.

16. The use of claim 15, wherein the RNA viruses include, but not limited to, coronaviruses, such as severe acute respiratory syndrome coronavirus (SARS-CoV), Middle East Respiratory Syndrome Coronavirus (MERSCoV) and 2019-nCoV; Ebola virus, bunya virus, avian influenza H9N2, H1N1, H7N9, arena virus, rabies virus, hepatitis C HCV, hepatitis B HBV, human immunodeficiency virus HIV-1, herpes simplex virus HSV-1, HSV-2, etc.

17. The use of claim 16, wherein the RNA viruses include, but not limited to: severe acute respiratory syndrome coronavirus (SARS-CoV), Middle East Respiratory Syndrome Coronavirus (MERS-CoV) and 2019-nCoV, Ebola virus, fever with thrombocytopenia syndrome virus (sftsv), avian influenza virus H9N2, H1N1.

18. A method for treating RNA virus infection, comprising administering a therapeutically effective amount of the compound of any one of claims 1-9 or the pharmaceutical composition of any one of claims 10, 11, 15-17 to a subject in need of the treatment for viral infections.
